# EUROPEAN PATENT APPLICATION

(11) **EP 1 938 867 A2**
(43) Date of publication of application: **02.07.2008**
(21) Application number: 07019176.2
(22) Date of filing: 28.09.2007
(51) Int. Cl.: A61Q 19/08, A61Q 19/02, A61Q 19/00, A61K 8/98, A61K 8/99, A61K 8/60, A61K 8/64

(54) **Compounding ingredients for basic cosmetics and basic cosmetics**

(30) Priority: 21.12.2006 JP 2006344970
(71) Applicant: Nissei Bio Co., Ltd., Hokkaido 061-1374 (JP)
(72) Inventor: Matsunaga, Masaji, Tokyo (JP); Yoshida, Fumito, Tokyo (JP); Mori, Takao, Sapporo-shi Hokkaido (JP)
(74) Representative: HOFFMANN EITLE

(57) **Abstract**

The present invention relates to a compounding ingredients for basic cosmetics and basic cosmetics containing the compounding ingredients which contain enzymes or hydrolysis products of nucleoprotein and/or DNA or RNA, or deoxy oligonucleotide, deoxy mononucleotide, oligopeptide, oligonucleotide mononucleotide separated from the hydrolysis products, or at least two kinds of mixtures selected from the hydrolysis products or compounds as active ingredients. Because the active ingredients such as deoxy oligonucleotide and others have comparatively small molecular weights, they are easy to be absorbed percutaneously, and when they are percutaneously absorbed, they exhibit cellular stimulating effects and blood circulation promoting effects, and therefore, in the event that they are applied to the facial skin, they marvelously prevent skin roughness, blotches, freckles, wrinkles, etc. and exhibit superb skin-lightening effects.

## Description

### BACKGROUND OF THE INVENTION

### Field of the Invention

The present invention relates to compounding ingredients for basic cosmetics and basic cosmetics, and more specifically, to a compounding ingredients for basic cosmetics and basic cosmetics containing the compounding ingredients which contain enzymes or hydrolysis products of nucleoprotein and/or DNA or RNA, or deoxy oligonucleotide, deoxy mononucleotide, oligopeptide, oligonucleotide, mononucleotide separated from the hydrolysis products, or at least two kinds of mixtures selected from the hydrolysis products or compounds as active ingredients.

### Description of the Related Art

Chapping of the skin, blotches, freckles, wrinkles etc. are assumed to be caused by various matters, effects of female hormone resulting from aging, stimulation of ultraviolet rays from sunlight, adverse effect of increased peroxide concentration in the body on epidermis, oversecretion of sebum, drop of blood flow to epidermis, malnutrition, mental stress, and the like.

It is manifested that chapping of the face skin, blotches, freckles, wrinkles, etc. are of a matter of serious concern for cosmetic reasons by an increase in product lineups of basic cosmetics not only for women but also for men in recent years. Because the face serves as a large element to give a first impression to other people, there are many persons who yearn for having a lustrous supple skin (beautiful skin) and a fair and clear skin (whitening), and therefore, interests of the world in general in basic skin cosmetics that improve chapping of the face Skin, blotches, freckles, wrinkles, etc. have been more and more growing.

In conventional cosmetics which are included in the category of "basic cosmetics," many efforts have been made to increase skin moisturizing action and to achieve lustrous skin by adding a moisturizing agent and oil component such as glycerin and the like. Recently, to achieve whitening effects to the skin, cosmetics containing L-ascorbic acid and its derivatives which have the action of suppressing the production of melanin, or hydroquinone derivatives have made an appearance.

And now, in recent years, to reflect growing interest of the world in general in health, healthfoods using deoxyribonucleic acid (DNA), ribonucleic acid (RNA) or nucleoprotein for material or active ingredients have been provided. It is proposed to depolymerize nucleoprotein of high molecular weight in order to make it water-soluble and easily-digestive (patent literature 1).

It is known that deoxyribonucleic acid (DNA), ribonucleic acid (RNA), or nucleoprotein has antiaging effects, but attempts to apply them to basic cosmetics have not yet been thoroughly made.

### [Patent Literature 1] JP-A-2004-16143

Chapping of the skin, blotches, freckles, wrinkles, and other skin troubles are caused by aged facial skin, poor circulation, influence of ultraviolet rays, stresses, and other various factors, but are directly caused by degraded metabolism of skin cells and decreased regenerative function of new cells.

Conventional basic cosmetics are not intended to work on the metabolism mechanism of skin cells and to exhibit their functions but are anything more than merely pursuing effects on the skin surface. Consequently, effects that can be said satisfactory have never been obtained for chapping of the skin, blotches, freckles, wrinkles, whitening and the like.

In addition, L-ascorbic acid and their derivatives which have functions to suppress melanin generation mentioned above are not stable and have insufficient ultraviolet ray inflammation prevention effects, and hydroquinone derivatives have a problem with safety. Consequently, it is difficult to directly expect the melanin biosynthesis prevention effects and melanin bleaching action which the above-mentioned compounds possess as active ingredients of cosmetics.

That is, products with high skin whitening effects which promote regeneration of new cells by cellular stimulation and blood flow facilitation and prevent chapping of the face skin, blotches, freckles, wrinkles, etc. are desired but conventional basic cosmetics have not yet achieved the effects that satisfy these requirements.

Consequently, it has been desired to develop new basic cosmetics which stimulate cells themselves and which produce lively skin.

### SUMMARY OF THE INVENTION

The present inventors devoted themselves to studies to obtain new basic cosmetics which provide prevention effects of chapping of the face skin, blotches, freckles, wrinkles, etc. and skin whitening effects, and as a result, found that decomposition products containing deoxy oligonucleotide, deoxy mononucleotide or oligopeptide obtained by enzyme-degradation treating or hydrolysis treating nucleoprotein and/or DNA, or decomposition products containing oligonucleotide or mononucleotide obtained by enzyme-degradation treating or hydrolysis-treating RNA, or their mixtures provide outstanding prevention effects of chapping of the face skin, blotches, freckles, wrinkles, etc., and have completed the present invention.

That is, the compounding ingredients for basic cosmetics according to the present invention are characterized by containing as active ingredients,
decomposition products obtained by depolymerizing by enzyme degradation or hydrolysis of nucleoprotein and/or DNA and containing 20 to 50% fractions of molecular weight ranging from 1000 to 3000, or deoxy oligonucleotide, deoxy mononucleotide or oligopeptide separated from the decomposition products; or
decomposition products obtained by depolymerizing by enzyme degradation or hydrolysis of RNA and containing 20 to 50% fractions of molecular weight ranging from 1000 to 3000, or oligonucleotide or mononucleotide separated from the decomposition products; or
at least two kinds of mixtures selected from the group consisting of the nucleoprotein and/or DNA decomposition products, the RNA decomposition products, deoxy oligonucleotide or mononucleotide, oligopeptide, oligonucleotide, and mononucleotide.

In a preferable embodiment of the compounding ingredients for basic cosmetics according to the present invention, it is preferable to obtain the nucleoproteins and DNA from soft roes of salmon, trout, herring, bonito, cod, and others.

In addition, the RNA is obtained from enzymes selected from the group consisting of beer yeast, torula yeast, milk yeast, and baker's yeast.

Furthermore, the basic cosmetics according to the present invention contain the above-mentioned compounding ingredients for basic cosmetics.

The basic cosmetics are preferably selected from the group consisting of lotions, milky lotions, creams, gels, jellies, extracts, lip creams, facial masks, or massage masks.

The compounding ingredients for basic cosmetics contain decomposition products obtained by depolymerizing by enzyme degradation or hydrolysis of nucleoprotein and/or DNA and containing 20 to 50% fractions of molecular weight ranging from 1000 to 3000, or deoxy oligonucleotide, deoxy mononucleotide, oligopeptide, oligonucleotide, mononucleotide separated from the decomposition products, or at least two kinds of mixtures selected from the decomposition products or the compounds as active ingredients. Because the deoxy oligonucleotide, etc. have comparatively small molecular weight, they are easy to be absorbed percutaneously and they provide cellular stimulation action and blood flow facilitation action when absorbed percutaneously. Consequently, in the event that they are applied to the epidermis of facial surface, they beautifully prevent chapping of the skin, blotches, freckles, wrinkles, etc., and achieve skin whitening effects.

In addition, because the basic cosmetics according to the present invention contain the compounding ingredients for basic cosmetics, when they are applied to epidermis of a face, effects of preventing chapping of the skin, blotches, freckles, wrinkles, etc. which the compounding ingredients for basic cosmetics possess are exhibited and skin whitening effects are achieved.

### BRIEF DESCRIPTION OF THE DRAWINGS

Fig. 1 is a diagram that shows an analysis example of deoxy oligonucleotide by HPLC of nuclease-treated DNA (decomposition product);
Fig. 2 is a diagram that shows an analysis example of deoxy oligonucleotide by HPLC of nuclease-treated nucleoprotein (decomposition product); and
Fig. 3 is a diagram that shows an analysis example of oligonucleotide by HPLC of nuclease-treated RNA (decomposition product).

### DETAILED DESCRIPTION OF THE INVENTION

Deoxy oligonucleotide or deoxy mononucleotide used as an active ingredient of the compounding ingredients for basic cosmetics of the present invention as well as for purified preparations are able to be obtained by enzyme-degradation treating or hydrolysis-treating DNA, and oligonucleotide or mononucleotide by enzyme-degradation treating or hydrolysis-treating RNA, and oligopeptide by enzyme-degradation treating or hydrolysis-treating nucleoprotein, respectively.

DNA and nucleoprotein are able to be obtained by, for example, extracting and purifying soft roes of fish. Examples of the fish include salmon, trout, herring, bonito, and cod, and in particular, salmon is preferable.

Now, DNA will be described more in detail as follows.

DNA which is the manufacturing material of the compounding ingredients for basic cosmetics of the present invention may be of various modes, and for example, may be double-stranded, single-stranded, or circular DNA. The DNA supply sources are various living organisms, such as animals, plants, microorganisms and the like. Testes (soft roes) of fishes which are wastes in fish-processing, in particular, salmon, trout, herring, bonito, and cod contain, in particular, a large amount of DNA, but conventionally, they were not effectively utilized as resources and a lot of them were discarded. Consequently, from the viewpoint of recycling of wastes, it is desirable to utilize the testes-derived DNA. In addition, it is possible to use DNA obtained from thymus glands of mammals and birds, for example, pigs, chickens and the like. Furthermore, synthetic DNA, too, may be able to be used.

By the way, to obtain DNA from fish soft roes, the extraction and purification method stipulated in Japanese Patent Application Laid-open No. 2005-245394.

Specifically, first of all, fish soft roes are coarsely ground, protein decomposition enzyme (protease) treatment is performed on coarsely ground fish soft roes under the conditions in which DNA is not decomposed, and the enzyme-treated solution is filtered. And using a hollow fiber membrane whose molecular weight cutoff ranges from 2,000 to 1,000,000, the filtrate is dialyzed to remove decomposed protein and ions and at the same time to concentrate DNA. Furthermore, DNA salts are allowed to be precipitated from the dialyzed solution or the solution is concentrated and these precipitates or concentrates are recovered.

The DNA salt powder obtained by drying the DNA salts obtained by the above method may be used for manufacturing material of the compounding ingredients for basic cosmetics of the present invention.

To obtain DNA from fish soft roes, not only this method but also publicly known methods may be used.

RNA is obtained from yeasts, and preferably, is obtained by extracting from a yeast selected from the group consisting of beer yeast, torula yeast, milk yeast, and baker's yeast and refining it.

The enzyme which treats DNA and RNA is, for example, nuclease, and for example, Penicillium-derived nuclease is preferable.

Oligopeptide is obtained by hydrolyzing nucleoprotein (protein constituent of cell nuclei) contained in fish soft roes, etc. by protease.

The protease primarily consists of trypsin. Trypsin is serine protease which has high specificity, and selectively hydrolyzes the peptide bond on the carboxyl side of arginine and lysine, and therefore, this is suited for hydrolyzing protamine which contains arginine. In addition, the above-mentioned protease may contain other protease, for example, chymotrypsin, etc. in addition to trypsin, too. For favorable protease, protease commercially available from Novozymes Japan Ltd. (former Novo Nordisk Bioindustry Ltd.) can be mentioned.

For the nuclease, 3', 5'-phosphodiester linkage of deoxyribonucleic acid (DNA) and ribonucleic acid (RNA) is hydrolyzed to generate 5'-(deoxy) nucleotide of oligomer polymerization. There is no particular limitation to the character of the nuclease but it is preferable to have a certain level of thermostability. This kind of nuclease is able to be obtained as a commercially-available product from Amano Enzyme Inc. (former Amano Pharmaceutical Co., Ltd.), Sigma Genosys Japan and the like.

What is important in hydrolysis treatment of DNA, RNA and nucleoprotein using the nuclease is the temperature at which reactions are conducted. The reaction temperature must be within the range from 60 to 75°C, and 70°C is most preferable. Allowing reactions to take place at temperatures lower than the relevant temperature range prevents depolymerization of DNA, RNA and nucleoprotein to satisfactorily take place, and the decomposition products do not acquire water solubility. On the other hand, allowing reactions to take place at temperatures higher than the relevant temperature range causes depolymerization to take place excessively, and there is a fear of losing the superb effects of nucleoprotein.

As described above, by treating DNA, RNA and nucleoprotein by hydrolysis conducted at 60 to 75°C using nuclease, it is possible to depolymerize DNA, RNA and nucleoprotein to such an extent that they contain 20 to 50% fractions whose molecular weight is 1000 to 3000, as well as to contain, for example, 30 to 50% fractions whose molecular weight is 1000 or less in a quantity larger than the quantity of fractions of molecular weight of 1000 to 3000, and by this, DNA, RNA and nucleoprotein decomposition products with both water solubility and transdermal absorptivity equipped can be manufactured.

In the nucleoprotein decomposition products and DNA decomposition products obtained by the foregoing treatment, depolymerized deoxy oligonucleotide, deoxy mononucleotide and oligopeptide are contained as a principal part or a large part, and insufficiently depolymerized deoxy nucleotide, etc. are contained as a negligible-amount part.

In addition, in the RNA decomposition product obtained by the similar treatment, oligonucleotide and mononucleotide are contained as a principal part or a large part, and insufficiently depolymerized nucleotide, etc. are contained as a negligible-amount part.

Consequently, almost all or the majority of nucleotides (deoxyoligonucleotide, deoxymononucleotide, oligonucleotide, and mononucleotide) are composed with monomers which originally do not take any helical strand, oligomers of perfect single-strand, and oligomers which have double-strand structure only partly. In other words, even if a case in which the above-mentioned depolymerization did not take place successfully is assumed, the ratio of double-strand of nucleotides contained in the decomposition products never exceeds 20%.

By the way, in order to obtain oligopeptide by hydrolyzing nucleoprotein, protease treatment must be performed, while in order to obtain oligonucleotide, nuclease treatment must be performed, but preferably, it is desirable to first treat with protease and then, treat with nuclease as a matter of operational convenience as well as from the viewpoint of the quality of final products obtained.

It is possible to use the decomposition product obtained by enzyme-decomposing or hydrolyzing nucleoprotein and/or DNA or the decomposition product obtained by enzyme-decomposing or hydrolyzing RNA as they are (without refining) for active ingredients of the compounding ingredients for basic cosmetics of the present invention. In the decomposition products, for example, amino acid, etc. may be contained.

In addition, it is possible to use the following compounds: deoxy oligonucleotide, deoxy mononucleotide, oligopeptide, oligonucleotide and mononucleotide, separated from nucleoprotein and/or DNA or RNA decomposition products by commonly-used separation means and/or refining means as active ingredients of the compounding ingredients for basic cosmetics of the present invention.

In such event, the strand length of deoxy oligonucleotide and oligo nucleotide contained in the nucleoprotein, DNA and RNA decomposition products or separated and refined by the use of commonly-used separation means and/or refining means from the decomposition products is preferably 2 to 12.

The decomposition products and the compounds may be used independently, or may be used by mixing at least two kinds of these decomposition products or compounds. In the event that the decomposition products and the compounds are mixed, the mixture ratio may be suitably selected.

In such event, it is particularly preferable that as the decomposition products and the compounds, at least any one of deoxy oligonucleotide or the olygonucleotide of strands 2 to 12 long is contained, and at the same time, a total of the deoxy oligonucleotide and the oligonucleotide of strands 2 to 12 long is 20% or more with respect to the grand total of the decomposition products and the compounds.

In addition, the decomposition products and the compounds may be used by further combining with commonly-used additive ingredients of the compounding ingredients for basic cosmetics at a predetermined ratio.

The concentration of active ingredients (the decomposition products and/or the compounds) in the compounding ingredients for basic cosmetics of the present invention may be suitably selected.

In addition, the basic cosmetics of the present invention contain the compounding ingredients for basic cosmetics.

The dosage form which the basic cosmetics according to the present invention can be suitably selected if it is a dosage form applicable to the skin. Preferably, basic cosmetics are desirable to be skin lotions, milky lotions, creams, gels, jellies, extracts, lip creams, facial masks, or massage masks.

The compounding ingredients for basic cosmetics according to the present invention may have publicly known ingredients which can be compounded to existing basic cosmetics in addition to the compounding ingredient for basic cosmetics. For example, perfumes, moisturizing agents, etc. may be compounded independently or in combination.

To the basic cosmetics of the present invention, as medication ingredients, Vitamin E or its derivatives, for example, Vitamin E acetate; vasodilator such as derivative of acetylcholine, etc.; skin function accelerating agent such as cepharanthine, etc.; glycyrrhetinic acid and its derivatives; female hormones such as estradiol, estrone, etc.; amino acids such as serine, methionine, arginine, etc.; Vitamin A, Vitamin B₁, Vitamin B₆, biotin, pantothenic acid or its derivatives, and other Vitamins may be compounded independently or in combinations.

Furthermore, to the basic cosmetics of the present invention, additives generally used for skin external preparations such as cosmetics, medicinal products, etc., for example, oil contents, antiseptics, surfactants, dispersion stabilizers, thickeners, moistening agents, ultraviolet absorbers, antioxidants, pH adjusters, purified water, alcohols, etc. may be compounded independently or in combinations.

### [Example]

In examples and comparisons shown as follows, the present invention will be described specifically and further in detail. The following examples are for explanation of the present invention only but the technological scope shall not be restricted by these examples.

The compounding amount in the following examples and comparisons shall be the percent in mass with respect to the total amount. In addition, the amount of prototype 1 through prototype 3 (containing decomposition products obtained by enzyme-decomposing DNA, DNA and nucleoprotein, or RNA, respectively) used in examples is shown as the amount of solid content.

### 1. Manufacture of (deoxy) olygonucleotide

For the salmon soft roe derived DNA, limited decomposition was performed using nuclease [for example, enzyme preparation nuclease "Amano" [available from Amano Enzyme (former Amano Pharmaceutical)], which is licensed as food additives. The produced deoxy mononucleotide and deoxy oligonucleotide were analyzed by an electrophoresis unit and optimum conditions were determined.

Specifically, to warm water adjusted to around 65°C, as material, DNA-Na salt powder was charged and stirred; then, the mixture was further heated to 70°C, nuclease was added in a suitable amount to a range from 0.05 to 0.25% with respect to the material and the mixture was allowed to react for 3 hours. Then, the mixture was heated at 85°C for 10 minutes to deactivate nuclease and then centrifuged, and to the supernatant, a spray dry method was applied, and dry powder (decomposition products) containing deoxy oligonucleotide was obtained.

### 2. Analysis of (deoxy) oligonucleotide

To warm water adjusted to around 65°C, as material, DNA-Na salt powder derived from salmon soft roes was charged and stirred; then, the mixture was further heated to 70°C, enzyme preparation nuclease '"Amano" [available from Amano Enzyme (former Amano Pharmaceutical)] was added 0.05% with respect to the material and the mixture was allowed to react for 3 hours and the decomposition product was obtained. Then, the mixture was heated at 85°C for 10 minutes to deactivate nuclease and then the decomposition product was analyzed by HPLC.

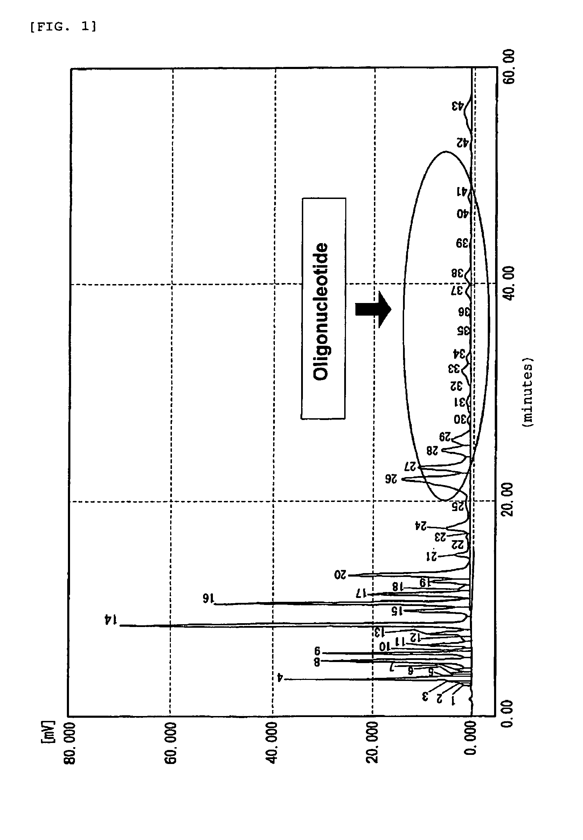

Fig. 1 shows an analysis example of decomposition product deoxy oligonucleotide by HPLC (high-performance liquid chromatography). In Fig. 1, 5'-deoxy mononucleotide and 3'-deoxy mononucleotide were eluted to peak 20, and the subsequent comparatively large peaks, that is, peaks after peak 26 can be regarded as absorption of deoxy oligonucleotide. In addition, peaks after peak 41 can be regarded as absorption of decomposition products whose molecular weight exceeds 3000. Consequently, as a result of computation from peak strengths of peak 26 through peak 41, it was clarified that in the present example, fractions of 31% deoxy oligonucleotide (molecular weight: 1000-3000) were contained with respect to the whole decomposition product.

### 3. Manufacture of basic cosmetics using (deoxy) oligonucleotide

As prototype 1 designated as dry powder (decomposition product) containing deoxy oligonucleotide obtained by the manufacturing method, using this prototype 1, the basic cosmetics of the present invention were manufactured as follows. For control, basic cosmetics which do not contain prototype 1 were manufactured. Table 1 summarizes these compositions.

### [Example 1]

Purified water was added to 95% ethanol, and to this, polyoxyethylene (25 mole), hardened castor oil ether, glycerin, and propylene glycol were added and stirred; then, prototype 1 was added and stirred to dissolve, and transparent liquid-form basic cosmetics of Example 1 was obtained.

### [Comparison 1]

Using the same amount of glycerin in place of prototype 1, basic cosmetics of Comparison 1 was obtained by the manufacturing method same as that of Example 1.

### 4. Blood flow volume measuring test

The basic cosmetics of Example 1 and the basic cosmetics of Comparison 1 were applied to the human upper arm at a rate of 10 µL, respectively, and 1 hour after the application, the blood flow volume was measured by the laser Doppler blood flow meter. The judgment of test results was performed in accordance with the following judgment criteria:
++: The blood flow volume markedly increased as compared to Comparison (marked effect)
+: The blood flow volume increased as compared to Comparison (effective)
+/-: The blood flow volume slightly increased as compared to Comparison (slightly effective)
-: The blood flow volume was equivalent to or less than Comparison (ineffective)

The results are summarized in Table 1 below.

### 5. Improvement test on chapping of the skin

With 10 middle-aged and older women who had chapping of the skin in legs designated as subjects, basic cosmetics of Example 1 and basic cosmetics of Comparison 1 were applied at about 1 g/once/day after taking bath at different places of right and left legs, respectively.

Based on the skin condition of the applied portions before the test and after 1 month, using the following judgment criteria, the test results were judged from the viewpoints of the skin peeling condition and moisture condition.

### 5-1 Judgment criteria of skin peeling and judgment of efficacy

The skin peeling conditions before and after the test were judged by the following judgment criteria:
0: No peeling; 1: Minor peeling; 2: Medium peeling; 3: Major peeling.

By the above judgment, the skin condition judgment improved by one stage from the condition before the test was designated as "slightly effective," and the skin condition judgment improved by two stages or more as "effective," and the skin condition on the same level or aggravated as "ineffective."

### 5-2 Judgment criteria of the moisture condition

The moisture condition of the skin after one month (moisture retention capacity) was measured by a moisture meter and judgment was made in accordance with the following criteria:

### <Judgment criteria of moisture condition>

++: The moisture retention capacity of the applied portion increased 5% or more before the test (marked effect)
+: The moisture retention capacity of the applied portion increased 2-5% before the test (effective)
+/-: The moisture retention capacity of the applied portion increased 0-2% before the test (slightly effective)
-: The moisture retention capacity of the applied portion was equivalent to or less than that before test (ineffective)

The results are summarized in Table 1 below.

### 6. Evaluation test

With middle-aged and older women as subjects, by the single blind study, the evaluation test was conducted on the basic cosmetics of Example 1 and the basis cosmetics of Comparison 1. By the way, 10 subjects were selected for each cosmetics. The evaluation was made by delivering questionnaires to subjects and answers were received on 5 items of "smoothness," "fine texture," "whitening condition," "elasticity of the skin," and "improved blotches and freckles" before the use and one month after the use. Based on the answers obtained, the test results were judged in accordance with the following judgment criteria:
++: Improved as compared to the condition before use (marked effect)
+: Slightly improved as compared to the condition before use (effective)
+/-: The condition was equivalent to or less than that before test (ineffective)

The results are summarized in Table 1 below.

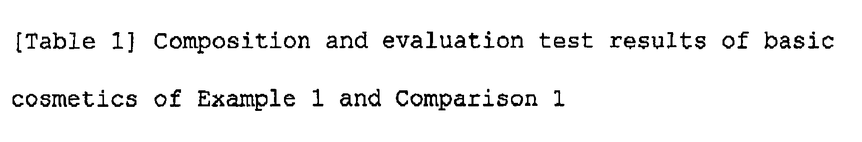

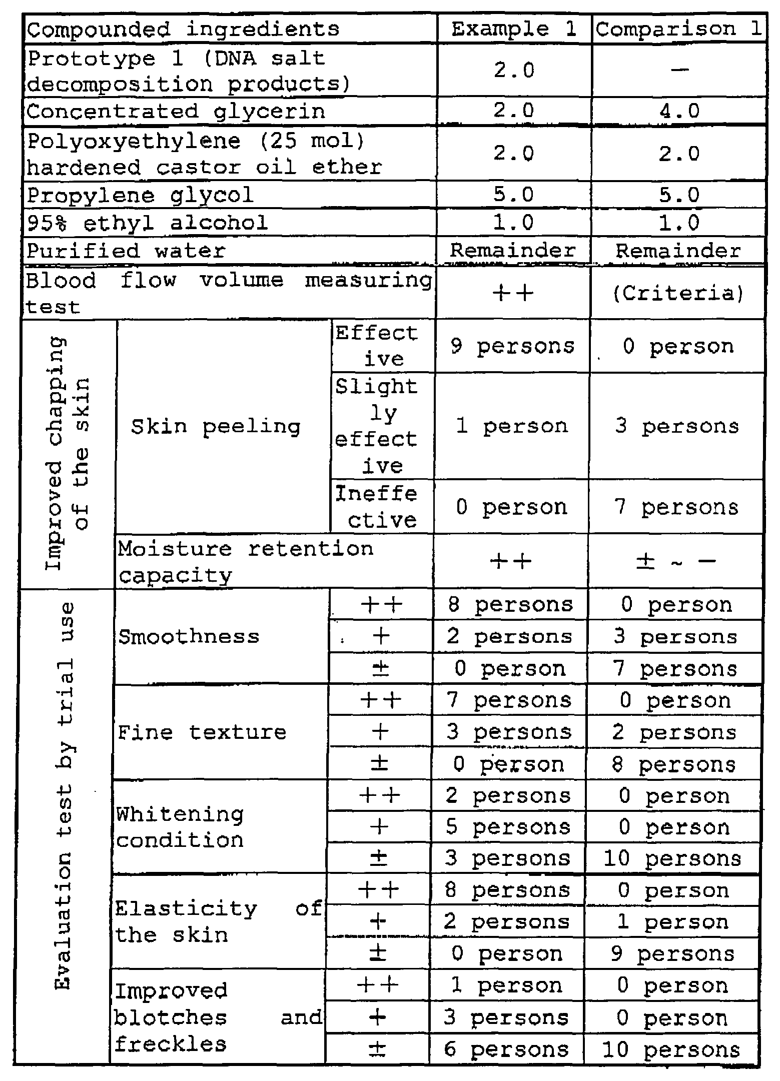

### 7. Manufacture and analysis of DNA and nucleoprotein decomposition products (prototype 2) or RNA decomposition products (prototype 3)

Products which contain as active ingredients the decomposition products obtained by enzyme-decomposing DNA and nucleoprotein manufactured by Nissei Bio Co., Ltd. were designated as prototype 2 (those containing deoxy oligonucleotide, deoxy mononucleotide and oligopeptide) and products which contain as active ingredients the decomposition products obtained by enzyme-decomposing RNA manufactured by Nissei Bio Co., Ltd. were designated as prototype 3 (those containing oligonucleotide and mononucleotide).

The manufacturing method and analysis results of prototype 2 and prototype 3 are shown as follows:

### 7-1 Manufacturing method and analysis of prototype 2 (DNA and nucleoprotein decomposition products

Prototype 2 was obtained by mixing the decomposition product obtained by depolymerizing DNA by enzyme decomposition treatment with the decomposition product obtained by depolymerizing nucleoprotein by enzyme decomposition treatment in an equal amount. The detail of the manufacturing method of each decomposition product is shown as follows:

### <DNA decomposition products>

### [Manufacturing method]

Manufacture was carried out by the method same as that of "1. Manufacture of (deoxy) olygonucleotide" mentioned above and DNA decomposition product was obtained.

### [Construction and composition]

Analysis was performed by the method same as that of "2. Analysis of (deoxy) olygonucleotide" mentioned above and it was found out that fractions of 31% deoxy olygonucleotide (molecular weight: 1000-3000) were contained with respect to the whole decomposition product.

### <Nucleoprotein decomposition products>

### [Manufacturing method]

To water, the nucleoprotein derived salmon soft roes (available from Nissei Bio Co., Ltd.), was charged and heated and stirred at 50°C; then, enzyme preparation protease "PTN" (available from Novozymes Japan) was added 0.065% and allowed to react for 4 hours; the mixture was further heated at 70°C and stirred. Then, enzyme preparation nuclease "Amano" (available from Amano Enzyme) 0.1% and allowed to react for 3 hours. Then, the mixture was heated at 85°C for 10 minutes to deactivate nuclease and then centrifuged, and by applying the spray dry method, dry powder (decomposition products) containing deoxy oligonucleotide was obtained.

### [Construction and composition]

The above-mentioned decomposition products obtained were analyzed by HPLC.

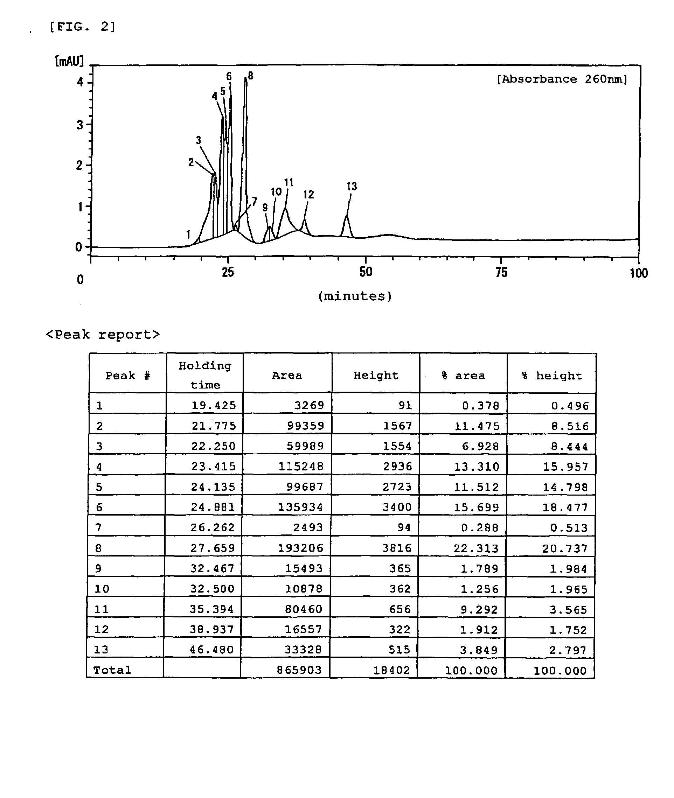

Fig. 2 shows an analysis example of deoxy olygonucleotide of decomposition products by HPLC (high-performance liquid chromatography) . In Fig. 2, peaks (peak 1 through peak 4) within holding time from 19 minutes to 24 minutes can be regarded as absorption of oligonucleotide. Consequently, as a result of computing from the peak strength of peak 1 through peak 4, in the present example, it was found out that fractions of 33.4% deoxy oligonucleotide (molecular weight: 1000-3000) were contained with respect to the whole decomposition products.

### 7-2. Manufacturing method and analysis of prototype 3 (RNA decomposition products)

Prototype 3 is the decomposition products obtained by depolymerizing RNA in place of material DNA by enzyme decomposition treatment by the manufacturing method as is the case with prototype 1, and the detail is shown as follows.

### <RNA decomposition products>

### [Manufacturing method]

To warm water adjusted to around 70°C, as material, RNA derived from enzyme (available from Nissei Bio Co., Ltd.) was charged and stirred; then, the mixture was further heated to 70°C, enzyme preparation nuclease "Amano" (available from Amano Enzyme) was added 0.05% with respect to the material and the mixture was allowed to react for 3 hours. Then, the mixture was heated at 85°C for 10 minutes to deactivate nuclease and then centrifuged, and by applying the spray dry method, dry powder (decomposition products) containing oligonucleotide was obtained.

### [Construction and composition]

The above-mentioned decomposition products obtained were analyzed by HPLC.

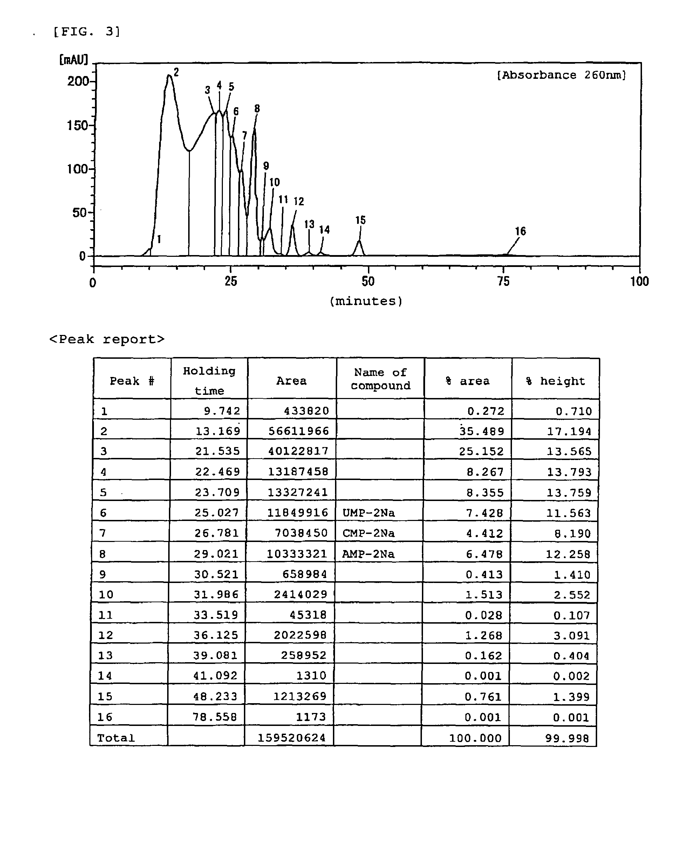

Fig. 3 shows an analysis example of olygonucleotide of decomposition products by HPLC (high-performance liquid chromatography). In Fig. 3, peaks (peak 2 through peak 5) within holding time from 13 minutes to 24 minutes can be regarded as absorption of oligonucleotide. Consequently, as a result of computing from the peak strength of peak 2 through peak 5, in the present example, it was found out that fractions of 41.1% oligonucleotide (molecular weight: 1000-3000) were contained with respect to the whole decomposition products.

### 8. Manufacture of various basic cosmetics

Using prototype 2 (DNA and nucleoprotein decomposition products) and prototype 3 (RNA decomposition products) obtained by the above-mentioned methods, basic cosmetics of the present invention were manufactured as follows. In addition, as controls, basic cosmetics of Comparison which did not contain any prototype 2 and prototype 3 were manufactured. Tables 2 through 5 summarize the compositions of these cosmetics.

By the way, suppliers of the products used for the following basic cosmetics are shown in parentheses.

### 8-1 Gel-form basic cosmetics

### Example 2 (Examples 2-1 through 2-3)

To purified water, Pemulen TR-1 (Nikko Chemicals Co., Ltd.) was gradually added with stirring and thoroughly dispersed, and then, Ultrez-10 (Nikko Chemicals) was stirred and dispersed, to which concentrated glycerin was added to obtain a gel base material. Separately, Lecinol WS-50 (Nikko Chemicals), jojoba oil, squalene, LIALCARB SR-1000 (Nikko Chemicals), AMITERMA-HD (Nihon-Emulsion Co., Ltd.) and rose hip oil were heated, dissolved, and mixed to have an oil-based ingredient. Furthermore, separately, Ceralipid W-2 (Nikko Chemicals), 1,3-butylene glycol (BG), dipropylene glycol (DpG) and 2-phenoxyethanol were added to superheat, stir, and mix (held at 300 rpm and 80°C for 5 minutes), and purified water was added and thoroughly stirred; then, the oil-based ingredient was combined and thoroughly mixed to obtain a compounded ingredient stock solution.

The gel base material and the compounded ingredient stock solution were mixed, to which ethanol was added; then, 18% sodium hydroxide was added to neutralize, stir, and mix. To this, prototype 2 and prototype 3, and an equivalent mixture of prototype 2 and prototype 3 (prototype 4), each mixed with purified water, were dissolved, respectively, and basic cosmetics of Example 2-1, Example 2-2, and Example 2-3 were obtained.

### Comparison 2:

Basic cosmetics of Comparison 2 was obtained in the method same as Example 2-1, Example 2-2, and Example 2-3 except for adding concentrated glycerin 2 mass% more to add a total of 7 mass% in place of prototype 2 and prototype 3.

### 8-2. Emulsified basic cosmetics

### Example 3 (Example 3-1 through 3-3)

To purified water, concentrated glycerin was added and the mixture was heated to 70°C and adjusted. Separately, to cetyl alcohol, beeswax, Vaseline, squalene, and dimethylpolysiloxane, monooleic acid ester, and glycerol monostearic acid ester were added and heated to 70°C. This was added to the water phase prepared in advance and preliminary emulsification was performed. Further, quince seed extract solution and ethanol were added and stirred, and the emulsified particles were homogenized by homomixer.

When this emulsified liquid reached 40°C, prototype 2 and prototype 3, and equivalent mixture of prototype 2 and prototype 3 (prototype 4), each mixed with purified water, were dissolved, respectively, further stirred, deaerated, filtered, and cooled, and emulsified basic cosmetics of Example 3-1, Example 3-2, and Example 3-3 were obtained.

### Comparison 3:

Basic cosmetics of Comparison 3 was obtained in the method same as Example 3-1, Example 3-2, and Example 3-3 except for adding concentrated glycerin 2 mass% more to add a total of 10 mass% in place of prototype 2 and prototype 3.

### 8-3. Lotion-form basic cosmetics (skin lotions)

### Example 4 (Examples 4-1 through 4-3)

In purified water, 1,3-butylene glycol, concentrated glycerin, buffering agent, and brown inhibitor were dissolved at room temperature. In addition, in ethanol, prototype 2 and prototype 3, and equivalent mixture of prototype 2 and prototype 3 (prototype 4), each mixed with oleyl alcohol, sorbitan monolaurate acid ester and purified water, were dissolved, respectively. These were mixed and stirred, and lotion-form basic cosmetics of Example 4-1, Example 4-2, and Example 4-3 were obtained.

### Comparison 4:

Lotion-form basic cosmetics of Comparison 4 was obtained in the method same as Example 4-1, Example 4-2, and Example 4-3 except for adding concentrated glycerin 2 mass% more to add a total of 6 mass% in place of prototype 2 and prototype 3.

### 8-4. Cream-form basic cosmetics

### Example 5 (Examples 5-1 through 5-3):

In purified water, 1, 3-butylene glycol, pentylene glycol, concentrated glycerin, and polyquaternium were successively dissolve, and heated to 80°C to have a water phase. On the other hand, polyglyceryl stearate, stearyl alcohol, behenyl alcohol, batyl alcohol, cetyl palmitate, glyceryl stearate, Crodaran SWL (Croda Japan KK), isopropyl palmitate, squalene, octyldodecyl myristate, macadamia nut oil, Trioctanoin, and dimethicone were mixed, and heated and stirred at 85°C to have an oil phase. To the water phase, the oil phase was injected and stirred (300 rpm), and was emulsified by homomixer for high viscosity (4000 rpm) . When this emulsified substance reaches 40°C, to purified water, prototype 2, prototype 3, and equivalent mixture of prototype 2 and prototype 3 (prototype 4), each mixed with sodium hydroxide, sodium citrate, and purified water, were dissolved, mixed and stirred, and cream-form basic cosmetics of Example 5-1, Example 5-2, and Example 5-3 were obtained.

### Comparison 5:

Cream-form basic cosmetics of Comparison 5 was obtained in the method same as Example 5-1, Example 5-2, and Example 5-3 except for adding concentrated glycerin 2 mass% more to add a total of 7 mass% in place of prototype 2 and prototype 3.

### 9. Performance test of basic cosmetics (gels, milky lotions, skin lotions, and creams)

For basic cosmetics of Example 2 through Example 5 and Comparison 2 through Comparison 5, the above-mentioned blood flow volume measuring test, improvement test on chapping of the skin, and evaluation test by trial use were performed, respectively, under the same conditions of Example 1 and Comparison 1.

Table 2 through Table 5 summarize the results.

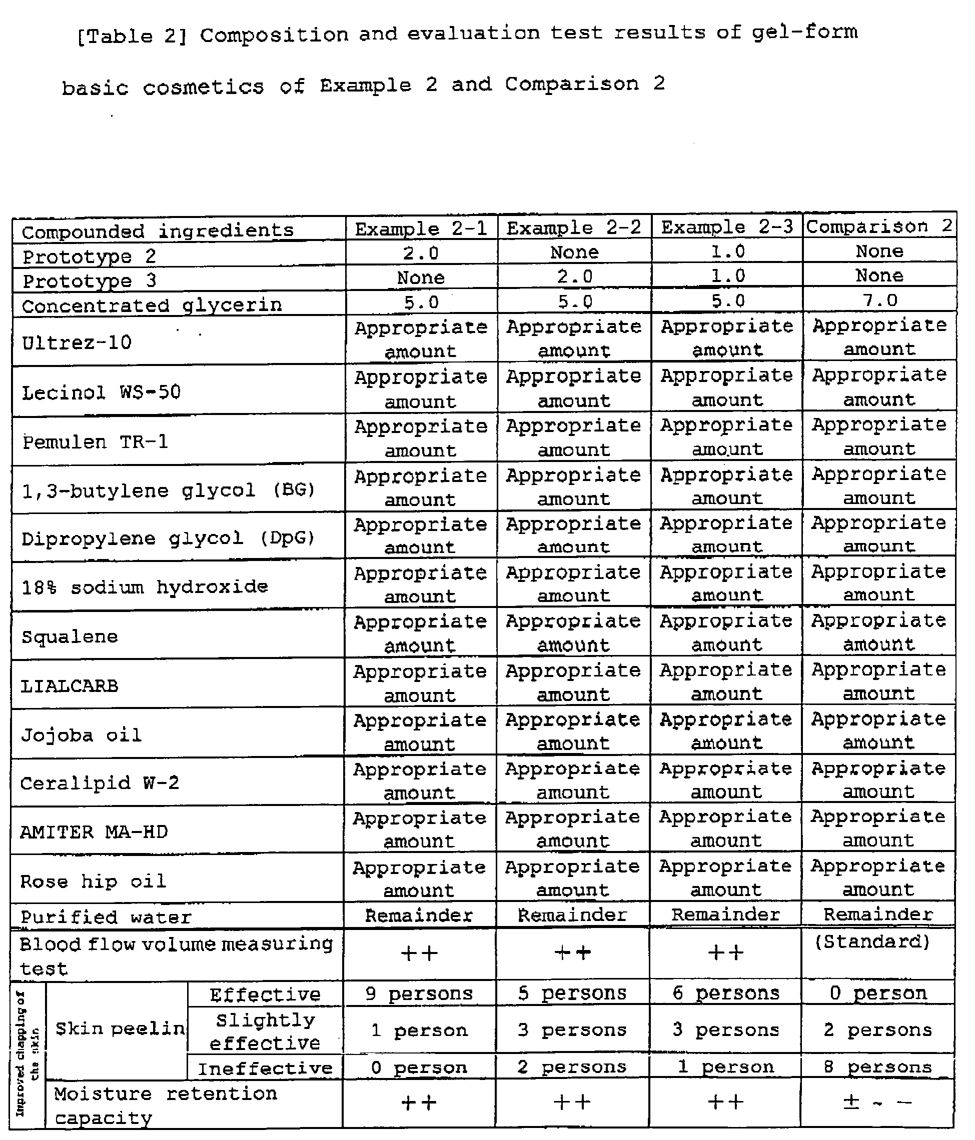

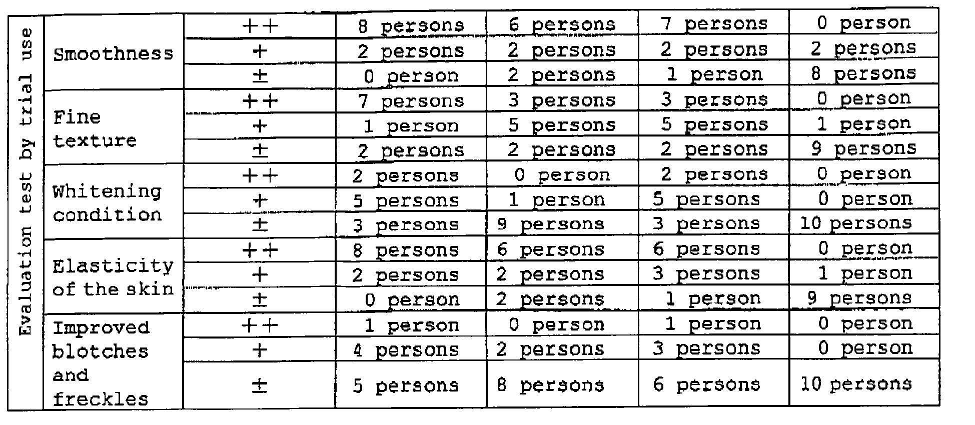

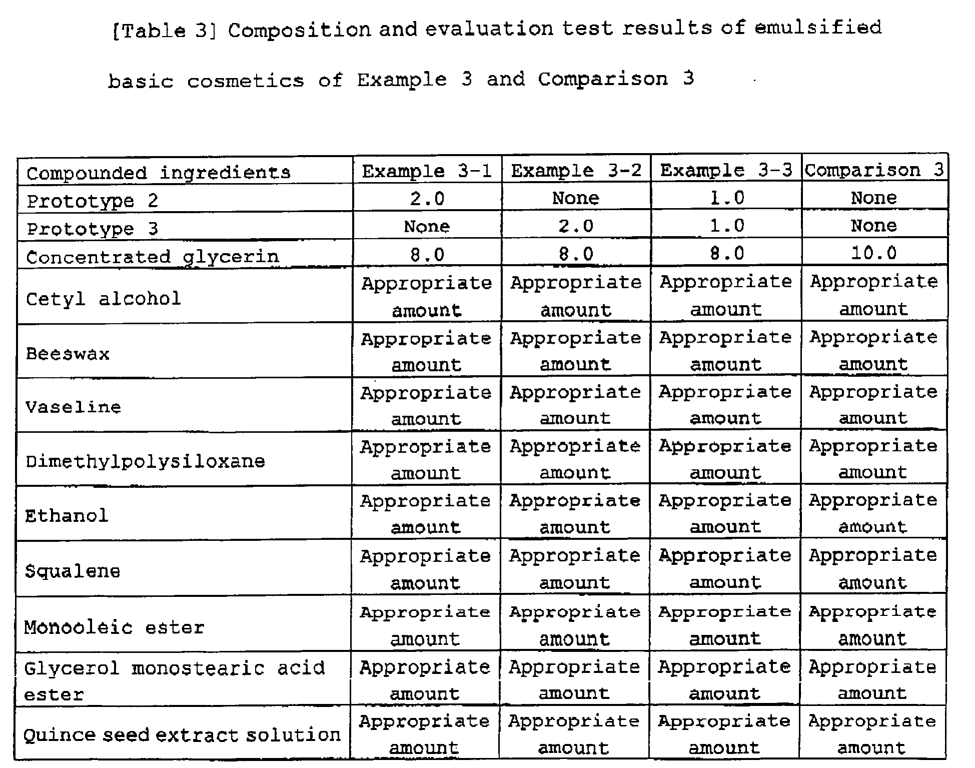

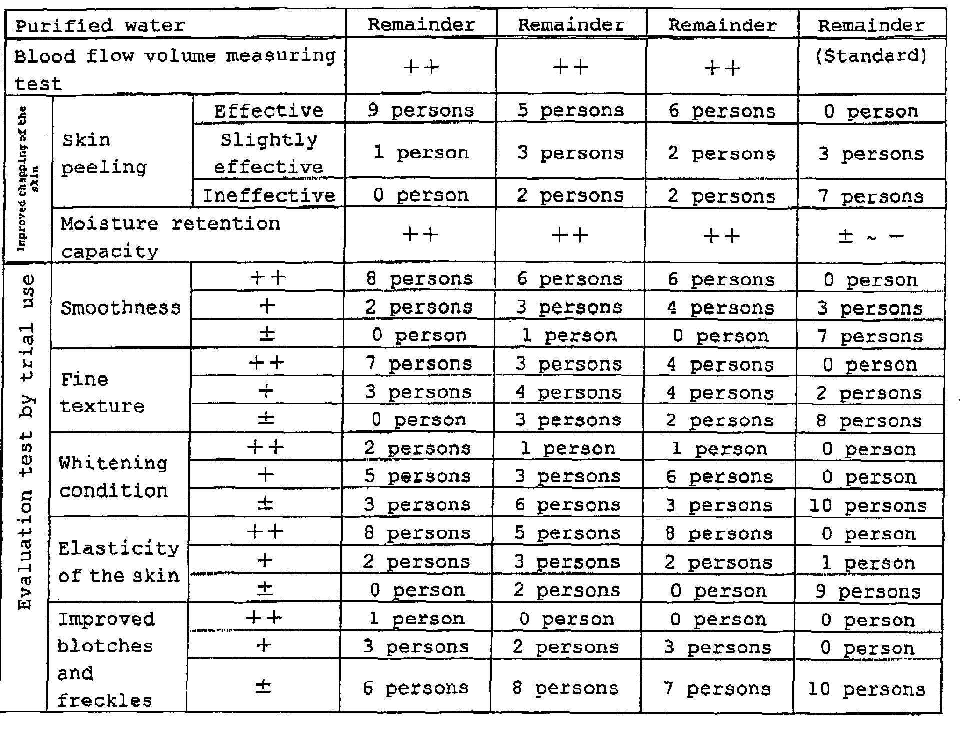

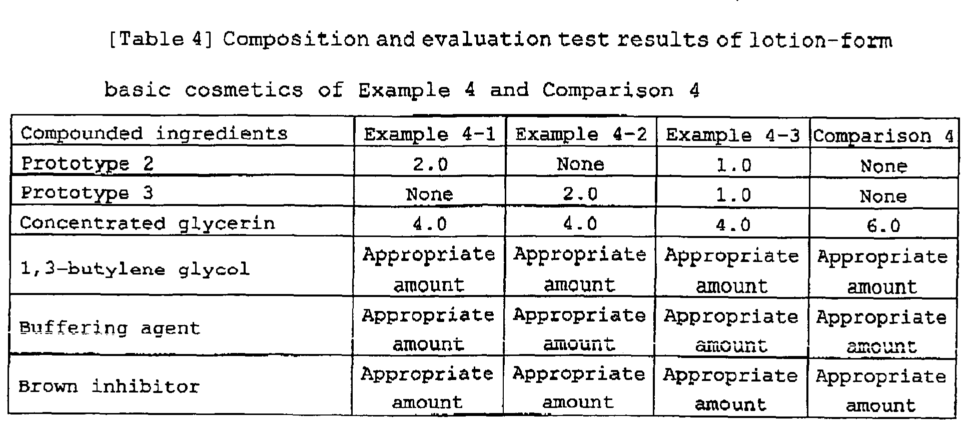

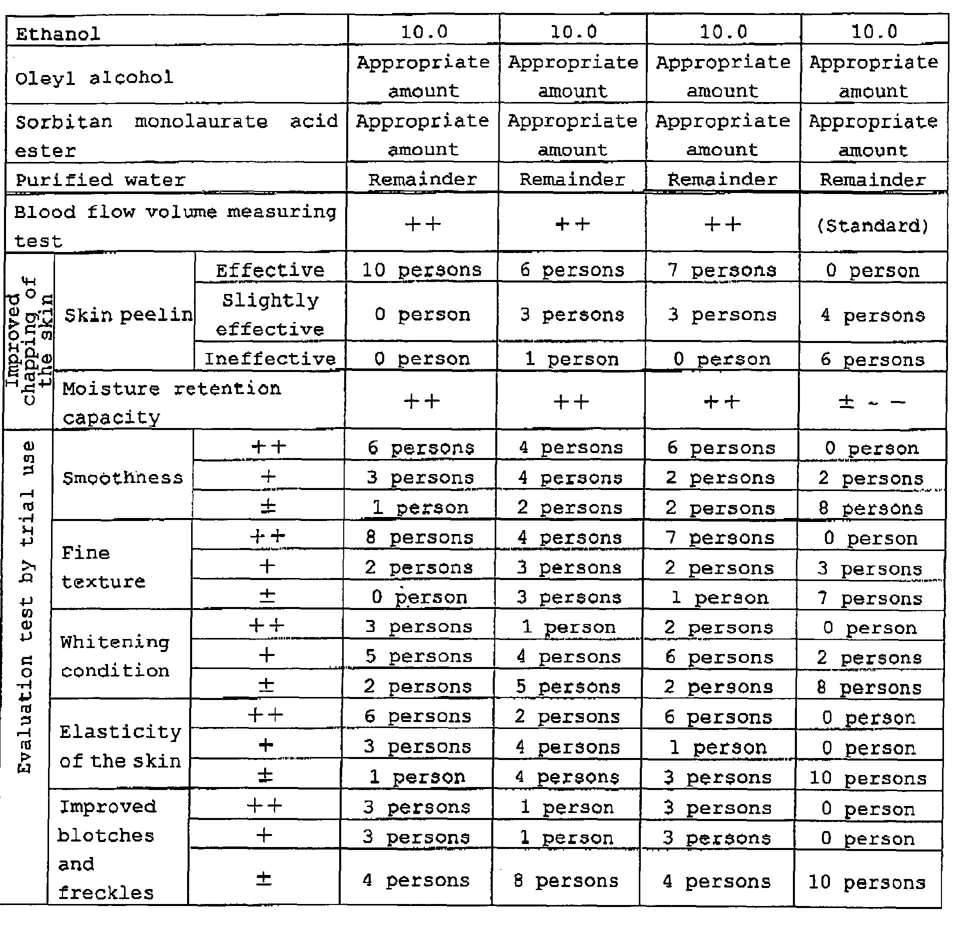

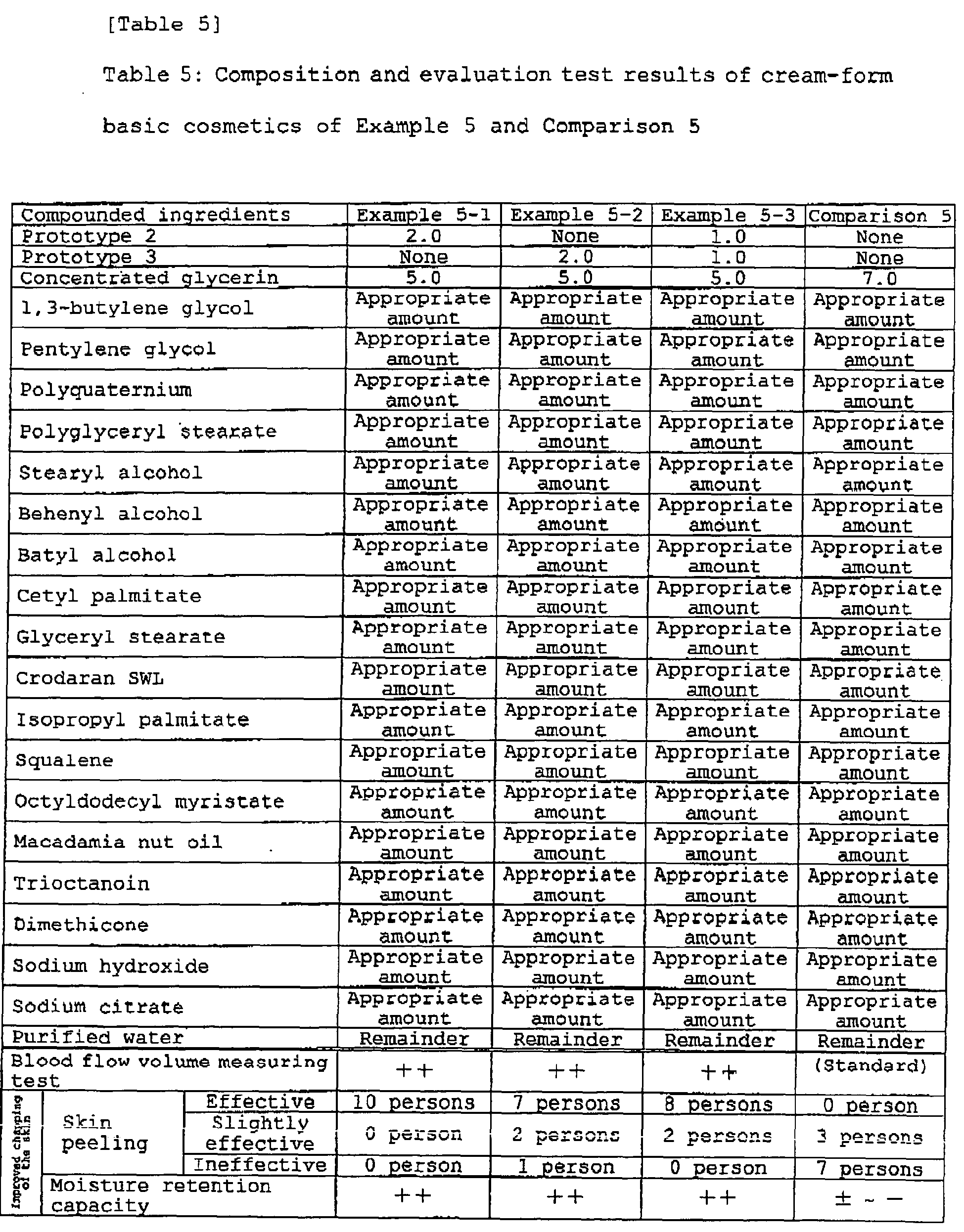

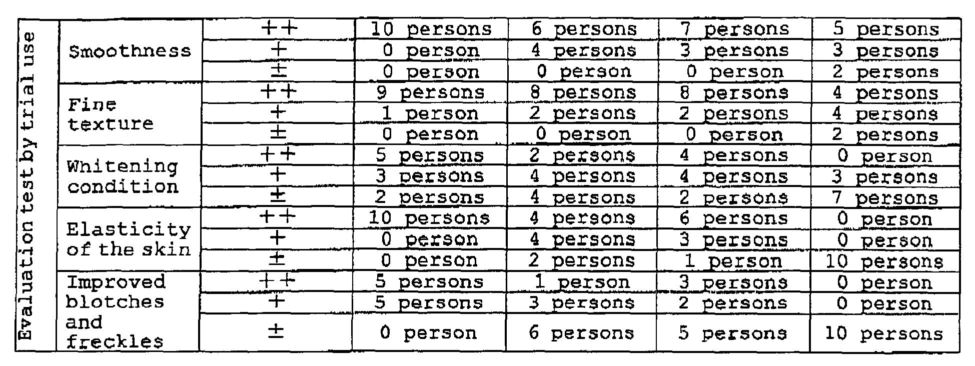

Based on the results shown in Table 1 and Table 2 though Table 5, the following were found out.
(1) The evaluations of blood flow volume measuring tests of Example 1 or Example 2 through Example 5 were ++ [the blood flow volume markedly increased as compared to Comparison (marked effect)] and it is apparent that the active ingredients of cosmetics of Example 1 through Example 5 penetrate from human skin and markedly increase the blood flow volume.
(2) In the improvement test on chapping of the skin of cosmetics of Example 1 or Example 2 through Example 5, cosmetics which contain Prototype 1, Prototype 2 and Prototype 4 that contains Prototype 2 exhibited marked efficacy, and the efficacy was confirmed with cosmetics which contain Prototype 3, too. By the way, for the left legs to which no skin care product was applied as controls, improvement of chapping of the skin was scarcely recognized, proving the efficacy of cosmetics which contain Prototype 1 through Prototype 4.
(3) In the test results shown in Table 1, the evaluation test by trial use of cosmetics by the cosmetics which contain Prototype 1 (DNA decomposition products) produced evaluation results in which marked improvement effects were recognized particularly in "smoothness," "fine texture," and "skin elasticity."
(4) In comparison of test results in the same cosmetics shown in Table 2 through Table 5, in the evaluation test by trial use of cosmetics containing Prototype 2 through Prototype 4 proved their superiority in any of skin care product forms in order of Prototype 2 > Prototype 4 > Prototype 3 > Comparison. In the cosmetics which contain Prototype 2 trough Prototype 4, marked important effects were recognized particularly in "smoothness" "fine texture" and "skin elasticity".
(5) On the other hand, in the evaluation test by trial use in Example 1 or Example 2 though Example 5, "whitening effects" and "improved blotches and freckles" were recognized with part of subjects, but with the skin metabolism, etc. taken into account, a slightly longer use would be required.

By the way, in Example 1 through Example 5, for active ingredients, prototype 1 (DNA salt decomposition products), prototype 2 (DNA and nucleoprotein decomposition products), prototype 3 (RNA decomposition products), and prototype 4 (mixture of prototype 2 and prototype 3) were used, but even when deoxy oligonucleotide, deoxy mononucleotide, oligopeptide, oligonucleotide, or mononucleotide separated from them and refined were used independently or in combination in place of prototype 1 through prototype 4, the effects were identified in the manner same as that in which prototype 1 through prototype 4 were used.

That is, based on the present embodiment, it has been determined that compounding ingredients for basic cosmetics which contain deoxy oligonucleotide, deoxy mononucleotide, oligopeptide, oligonucleotide or mononucleotide as well as basic cosmetics that contain the compounding ingredients of basic cosmetics have effects of increasing the blood flow volume, effects of improving chapping of the skin, as well as effects of improving smoothness of the skin, fine texture, and skin elasticity.

The formulation examples of basic cosmetics using Prototype 1 through Prototype 4 are shown as Example 6 through Example 8 as follows. By the way, the "prototype (s)" stipulated in these examples should mean any of the prototypes selected from Prototype 1 through Prototype 3, independently, respectively, and the equivalent mixture of Prototype 2 and Prototype 3 (Prototype 4).

In any of the examples, it was identified that the examples have effects of increased blood flow volume and effects of improving chapping of the skin, as well as effects of improving skin smoothness, fine texture, and skin elasticity. Example 6: lip cream

**[Table 6]**

| Formulation of lip cream | |
|---|---|
| Compounded ingredients | Blending quantity (mass%) |
| Solid paraffin | 10.0 |
| Caster oil | 20.4 |
| Lanolin | 14.0 |
| Beeswax | 5.0 |
| Candelilla wax | 12.0 |
| Carnauba wax | 7.0 |
| 2- cetyl ethylhexanoate | 18.0 |
| Isopropyl myristate | 12.0 |
| Prototype(s) | 1.5 |
| Perfume | 0.1 |

### <Manufacturing method>

Of the above-mentioned compounded ingredients, ingredients other than perfume were mixed and dissolved; then, perfume was added, stirred, and mixed. The mixture was injected in a mold and cooled to prepare lip cream.

### Example 7: Cold cream

**[Table 7]**

| Formulation of cold cream | |
|---|---|
| Compounded ingredients | Blending quantity (mass%) |
| Solid paraffin | 5.0 |
| Beeswax | 10.0 |
| Vaseline | 15.0 |
| Liquid paraffin | 41.0 |
| Glyceryl monostearate | 2.0 |
| Polyoxyethylene (20 mol) sorbitan monolaurate | 2.0 |
| Prototype(s) | 1.0 |
| Soap powder | 0.1 |
| Perfume | Appropriate quantity |
| Antiseptics | Appropriate quantity |
| Antioxidan | Appropriate quantity |
| Purified water | Remainder |

### <Manufacturing method>

To purified water, prototype(s) and soap powder were added, heated and dissolved, and kept to 70°C (water phase). Other ingredients were mixed, heated, and dissolved, and kept to 70°C (oil phase). Oil phase was gradually added to water phase with stirring, and after completing the addition, the mixture was homogeneously emulsified by homomixer, and after emulsification, the mixture was cooled to 30°C with good stirring, and cold cream was prepared.

### Example 8: clay facial mask

**[Table 8]**

| Formulation of clay facial mask | |
|---|---|
| Compounded ingredients | Blending quantity (mass%) |
| Prototype(s) | 1.0 |
| Kaolin | 25.0 |
| Ethanol | 5.0 |
| 1,3-butylene glycol | 10.0 |
| Glycerin | 5.0 |
| PEG-20 methyl glucose sesquistearate | 4.0 |
| Xanthan gum | 0.1 |
| Antiseptics | Appropriate quantity |
| Purified water | Remainder |

### <Manufacturing method>

1,3-butylene glycol, glycerin, PEG-20 methyl glucose sesquistearate, xanthan gum, antiseptics, and purified water were added and stirred to homogenize the whole.

While this is being stirred by homomixer, prototype(s) and kaolin were added, and the whole was homogenized, and further ethanol was added, stirred, and mixed, and clay facial masks were prepared.

## Claims

1. A compounding ingredients for basic cosmetics containing as active ingredients:
decomposition products obtained by depolymerizing by enzyme degradation or hydrolysis of nucleoprotein and/or DNA and containing 20 to 50% fractions of molecular weight ranging from 1000 to 3000, or deoxy oligonucleotide, deoxy mononucleotide or oligopeptide separated from the decomposition products; or
decomposition products obtained by depolymerizing by enzyme degradation or hydrolysis of RNA and containing 20 to 50% fractions of molecular weight ranging from 1000 to 3000, or oligonucleotide or mononucleotide separated from the decomposition products; or
at least two kinds of mixtures selected from the group consisting of the nucleoprotein and/or DNA decomposition products, the RNA decomposition products, deoxy oligonucleotide or mononucleotide, oligopeptide, oligonucleotide, and mononucleotide.

2. The compounding ingredients for basic cosmetics according to claim 1, wherein the nucleoproteins and DNA are obtained from soft roes of salmon, trout, herring, bonito, cod, and others.

3. The compounding ingredients for basic cosmetics according to claim 1, wherein the RNA is obtained from enzymes selected from the group consisting of beer yeast, torula yeast, milk yeast, and baker's yeast.

4. Basic cosmetics containing the compounding ingredients according to claims 1 through 3.

5. Basic cosmetics according to claim 4, wherein the basic cosmetics are selected from the group consisting of lotions, milky lotions, creams, gels, jellies, extracts, lip creams, facial masks, or massage masks.
